# EUROPEAN PATENT SPECIFICATION

(11) **EP 3 432 269 B1**
(45) Date of publication and mention of the grant of the patent: **21.10.2020**
(21) Application number: 17766019.8
(22) Date of filing: 11.01.2017
(51) Int. Cl.: G06T 7/20, C12M 1/34, G01N 33/50

(54) **INFORMATION PROCESSING DEVICE, INFORMATION PROCESSING METHOD, PROGRAM, AND INFORMATION PROCESSING SYSTEM**
INFORMATIONSVERARBEITUNGSVORRICHTUNG, INFORMATIONSVERARBEITUNGSVERFAHREN, PROGRAMM UND INFORMATIONSVERARBEITUNGSSYSTEM
DISPOSITIF DE TRAITEMENT D'INFORMATIONS, PROCÉDÉ DE TRAITEMENT D'INFORMATIONS, PROGRAMME, ET SYSTÈME DE TRAITEMENT D'INFORMATIONS

(30) Priority: 15.03.2016 JP 2016051054
(43) Date of publication of application: 23.01.2019
(73) Proprietor: Sony Corporation, Tokyo 108-0075 (JP)
(72) Inventor: OSHIMA, Shiori, Tokyo 108-0075 (JP); MATSUI, Eriko, Tokyo 108-0075 (JP)
(74) Representative: D Young & Co LLP
(86) International application number: PCT/JP2017/000690
(87) International publication number: WO 2017/159011

(56) References cited:
- WO-A1-2014/204993
- WO-A2-2012/137451
- JP-A- 2011 185 734
- JP-A- 2015 523 577
- JEREMY CHANGYU YEO ET AL: "High-throughput quantification of early stages of phagocytosis", BIOTECHNIQUES RAPID DISPATCHES, vol. 55, no. 3, 1 September 2013 (2013-09-01), XP055541974, US ISSN: 0736-6205, DOI: 10.2144/000114075 & Jeremy Changyu Yeo ET AL: "Supplementary Material For: High-throughput quantification of early stages of phagocytosis", BioTechniques, 1 September 2013 (2013-09-01), pages 1-8, XP055542467, Retrieved from the Internet: URL:https://www.future-science.com/doi/10. 2144/000114075 [retrieved on 2019-01-15]

## Description

### Technical Field

The present disclosure relates to an information processing device, an information processing method, a program, and an information processing system.

### Background Art

In the field of medical and life sciences, various kinds of functions expressed by biological samples have been evaluated. For example, a technology for evaluating a function related to absorption or release by a biological sample such as a phagocytosis function of phagocytes has been developed.

Specifically, the Non-Patent Literature 1 mentioned below discloses a technology for evaluating a phagocytosis function of phagocytes using flow cytometry and fluorescence imaging.

### Citation List

### Non-Patent Literature

Non-Patent Literature 1: D. H. Munn et.al. "Phagocytosis of Tumor Cells by Human Monocytes Cultured in Recombinant Macrophage Colony-Stimulating Factor," J. Exp. Med., Vol. 172(1990) p. 231 to 237.

JEREMY CHANGYU YEO ET AL: "High-throughput quantification of early stages of phagocytosis", BIOTECHNIQUES RAPID DISPATCHES, vol. 55, no. 3, 1 September 2013 (2013-09-01), XP055541974, ISSN: 0736-6205, DOl: 10.2144/000114075, pp.1-8 discloses an information processing device/system and corresponding method and program comprising: a detection unit configured to detect at least one region of interest in at least one captured image among a plurality of captured images of phagocytosis in a biological sample having different imaging times; a feature amount calculation unit configured to calculate a feature amount related to a change in the at least one region of interest in the plurality of captured images and an evaluation value calculation unit configured to calculate an evaluation value for a function related to absorption or release of the biological sample on a basis of the feature amount (quantitation of beads during the three stages of phagocytosis using an automated script which detects quantitative changes in three stages of phagocytosis).

### Patent Literature:

WO 2012/137451 discloses an information processing apparatus for searching corresponding regions of region of interest in e.g. MRI image, which involves colouring of contour lines.

WO 2014/204993 discloses a method for multimodal image sensing for region of interest capture involving a gating sensor.

JP2011185734 discloses an image evaluating apparatus for monitoring secretion by cells. JP2015523577 discloses a system for image analysis and measurement of biological samples, has sample holder configured so that light from illumination source simultaneously provides both epi-illumination and trans-illumination to sample.

### Disclosure of Invention

### Technical Problem

However, it is difficult to evaluate expression of the phagocytosis function of phagocytes being cultured in detail by using the flow cytometry technique. In addition, it is difficult to quantitatively evaluate the phagocytosis function simply by using fluorescence imaging.

Therefore, the present disclosure proposes a novel and improved information processing device, information processing method, program, and information processing system that can evaluate the function related to absorption or release of a biological sample in more detail.

### Solution to Problem

According to the present disclosure, there is provided an information processing device according to claim 1

In addition, according to the present disclosure, there is provided an information processing method according to claim 10.

In addition, according to the present disclosure, there is provided a program according to claim 11.

In addition, according to the present disclosure, there is provided an information processing system including: an imaging device including an imaging unit configured to generate a plurality of captured images of a biological sample having different imaging times; and an information processing device including a detection unit configured to detect at least one region of interest in at least one captured image among the plurality of captured images, a feature amount calculation unit configured to calculate a feature amount related to a change in the at least one region of interest in the plurality of captured images, and an evaluation value calculation unit configured to calculate an evaluation value for a function related to absorption or release of the biological sample on a basis of the feature amount.

### Advantageous Effects of Invention

According to the present disclosure described above, it is possible to evaluate the function related to absorption or release of a biological sample in more detail.

Note that the effects described above are not necessarily limitative. With or in the place of the above effects, there may be achieved any one of the effects described in this specification or other effects that may be grasped from this specification.

### Brief Description of Drawings

[FIG. 1] FIG. 1 is a diagram illustrating an overview of a configuration of an information processing system according to an embodiment of the present disclosure.
[FIG. 2] FIG. 2 is a functional block diagram illustrating a functional configuration example of an information processing device according to the embodiment of the present disclosure.
[FIG. 3] FIG. 3 is a diagram for describing a detection process of a region of interest performed by a detection unit according to the embodiment.
[FIG. 4] FIG. 4 is a diagram for describing an identification process of regions of interest performed by the detection unit according to the embodiment.
[FIG. 5] FIG. 5 is a diagram for describing a change in a position of a contour line of a region of interest.
[FIG. 6] FIG. 6 is a diagram for describing a change in a shape of a contour line of a region of interest.
[FIG. 7] FIG. 7 is a diagram for describing an internal motion of a region of interest.
[FIG. 8] FIG. 8 is a diagram for describing internal pixel information of a region of interest.
[FIG. 9] FIG. 9 is a graph illustrating an example of temporal change data of feature amounts calculated for an observation object.
[FIG. 10] FIG. 10 is a diagram illustrating an example of a gating process performed by an evaluation value calculation unit according to the embodiment.
[FIG. 11] FIG. 11 is a diagram for describing an example of control of a display mode by a display control unit based on a result of the gating process according to the embodiment.
[FIG. 12] FIG. 12 is a flowchart illustrating an example of a process performed by an information processing device according to the embodiment.
[FIG. 13] FIG. 13 illustrates examples of graphs showing temporal changes of feature amounts of a first region and a second region according to a modification example of the embodiment.
[FIG. 14] FIG. 14 is a block diagram illustrating a hardware configuration example of an information processing device according to an embodiment of the present disclosure.

### Mode(s) for Carrying Out the Invention

Hereinafter, (a) preferred embodiment(s) of the present disclosure will be described in detail with reference to the appended drawings. Note that, in this specification and the appended drawings, structural elements that have substantially the same function and structure are denoted with the same reference numerals, and repeated explanation of these structural elements is omitted.

Note that description will be provided in the following order.
1. Overview of information processing system
2. Information processing device
2.1. Configuration example
2.2. Process example
2.3. Effect
2.4. Modification example
3. Hardware configuration example
4. Conclusion

### <<1. Overview of information processing system>>

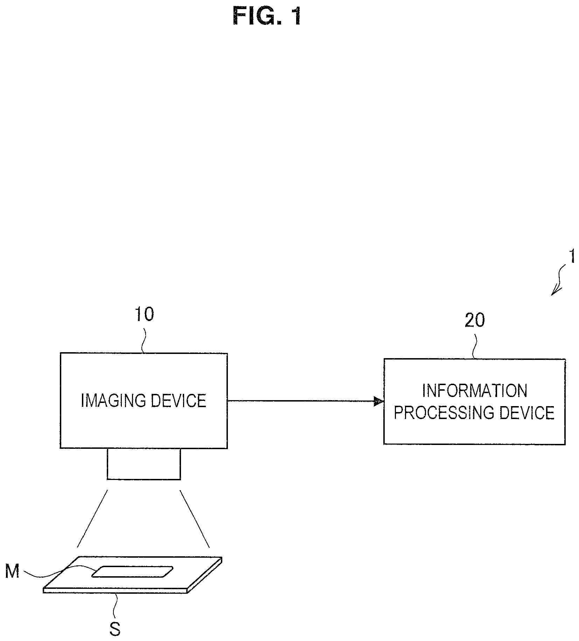

FIG. 1 is a diagram showing an overview of a configuration of an information processing system 1 according to an embodiment of the present disclosure. As shown in FIG. 1, the information processing system 1 is provided with an imaging device 10 and an information processing device 20. The imaging device 10 and the information processing device 20 are connected to each other via various types of wired or wireless networks.

### (Imaging device)

The imaging device 10 is a device which generates captured images (moving images). The imaging device 10 according to the present embodiment is realized by, for example, a digital camera. In addition, the imaging device 10 may be realized by any type of device having an imaging function, for example, a smartphone, a tablet, a game device, or a wearable device. The imaging device 10 images real spaces using various members, for example, an image sensor such as a charge coupled device (CCD) or a complementary metal oxide semiconductor (CMOS), a lens for controlling formation of a subject image in the image sensor, and the like. The image sensor and the various members realize the function of the imaging device 10 as an imaging unit. In addition, the imaging device 10 includes a communication device for transmitting and receiving captured images and the like to and from the information processing device 20. In the present embodiment, the imaging device 10 is provided above an imaging stage S to image a culture medium M in which a biological sample that is an observation object is cultured. In addition, the imaging device 10 generates moving image data by imaging the culture medium M at a specific frame rate. Note that the imaging device 10 may directly image the culture medium M (without involving another member), or may image the culture medium M via another member such as a microscope. In addition, although the frame rate is not particularly limited, it is desirable to set the frame rate according to the degree of a change of the observation object. Note that the imaging device 10 images a given imaging region including the culture medium M in order to accurately track a change of the observation object. Moving image data generated by the imaging device 10 is transmitted to the information processing device 20. Note that captured images generated by the imaging device 10 are not limited to moving images. Captured images generated by the imaging device 10 may be a plurality of captured images generated in plural at different imaging times. The plurality of captured images may be, for example, sequentially captured still images.

Note that, although the imaging device 10 is assumed to be a camera installed in an optical microscope or the like in the present embodiment, the present technology is not limited thereto. For example, the imaging device 10 may be an imaging device included in an electronic microscope using electron beams such as a scanning electron microscope (SEM) or a transmission electron microscope (TEM), or an imaging device included in a scanning probe microscope (SPM) that uses a short hand such as an atomic force microscope (AFM) or a scanning tunneling microscope (STM). In this case, a moving image generated by the imaging device 10 is a moving image obtained by irradiating the observation object with electron beams in the case of an electronic microscope. In addition, when the imaging device 10 is an SPM, a moving image generated by the imaging device 10 is a moving image obtained by tracing an observation object using a short hand. These moving images can also be analyzed by the information processing device 20 according to the present embodiment.

### (Information processing device)

The information processing device 20 is a device having an image analyzing function. The information processing device 20 is realized by any type of device having an image analyzing function such as a personal computer (PC), a tablet, or a smartphone. The information processing device 20 includes a processing circuit and a communication device. In the information processing device 20 according to the present embodiment, for example, the communication device acquires a plurality of captured images having different imaging times (e.g., moving images, sequentially captured still images, or the like) from the imaging device 10, and the processing circuit detects at least one region of interest in at least one captured image among the acquired plurality of captured images. Then, the processing circuit calculates a feature amount on the basis of a change in the detected region of interest appearing on the plurality of captured images. Then, the processing circuit calculates an evaluation value of a function related to absorption or release of phagocytes or the like (e.g., phagocytosis function) on the basis of the calculated feature amount. The processes performed by the processing circuit of the information processing device 20 are output to a storage device, a display device, or the like provided inside or outside the information processing device 20. Note that the information processing device 20 may be realized by one or a plurality of information processing devices on a network. A functional configuration for realizing the respective functions of the information processing device 20 will be described below.

Note that, although the information processing system 1 is constituted with the imaging device 10 and the information processing device 20 in the present embodiment, the present technology is not limited thereto. For example, the imaging device 10 may perform the processes of the information processing device 20 (e.g., a detection process and an analysis process). In this case, the information processing system 1 can be realized by the imaging device having the detection function and the analysis function.

Here, an observation object of the information processing system 1 according to the present embodiment will be described. First, an observation object according to the present embodiment is mainly a biological sample. A biological sample means, for example, an organism that can be observed using an optical microscope or the like including any of various cells, cell organelles, biological tissues, living bodies such as microorganisms, plankton, or the like, and an object having a biological function such as viruses. Particularly, a biological sample according to the present embodiment means a living body that can move in a culture medium M on an imaging stage S of the imaging device 10. Such a biological sample will be referred to as an observation object below.

An observation object according to the present embodiment is an observation object that expresses a function related to absorption or release. The function related to absorption may be, for example, a function of absorbing a substance from outside of a biological sample such as a phagocytosis function of a phagocyte. In this case, the phagocyte may be, for example, migratory immune cells such as macrophage cells, dendritic cells, or neutrophils. Such immune cells have a function of phagocytizing pathogens such as cancer cells, particles such as microbeads, bacteria, viruses, or the like. That is, these pathogens, particles, bacteria, or viruses correspond to substances to which the function of a biological sample is applied.

In addition, the function related to release may be, for example, a function of releasing a substance from the inside of a biological sample such as cell secretion. More specifically, the function related to release may be a function of releasing adenosine triphosphate (ATP) from a cell, a compound such as histamine, proteins such as enzymes, fine particles, calcium ions, or the like. That is, such compounds, proteins, fine particles, calcium ions, or the like correspond to substances to which the function of a biological sample is applied.

In addition, an observation object according to the present embodiment is not limited to a biological sample. For example, a non-biological sample such as a device such as a micro-electromechanical system (MEMS) having a substance absorption function, release function, or the like can be a target of an observation object according to the present embodiment.

As a technology of evaluating a function related to absorption or release of such an observation object, for example, there is the technology disclosed in the above-described Non-Patent Literature 1 (D. H. Munn et.al., "Phagocytosis of Tumor Cells by Human Monocytes Cultured in Recombinant Macrophage Colony-Stimulating Factor," J. Exp. Med., Vol. 172 (1990) p.231 to 237) using flow cytometry or fluorescence imaging. According to this technology, by performing flow cytometry on each of phagocytes that are observation objects, the presence or absence of expression of the phagocytosis function by the phagocytes can be evaluated. In addition, states of the phagocytosis function of the phagocytes can be qualitatively observed using fluorescence imaging.

However, the technology disclosed in the Non-Patent Literature is not sufficient for the function of an observation object related to absorption or release. In the evaluation of the phagocytosis function using flow cytometry, for example, the phagocytosis function of phagocytes is evaluated only at a specific timing (e.g., a timing at which the phagocytes are cultured in a culture medium for a predetermined period and the phagocytes are taken out from the culture medium). Thus, in the evaluation using flow cytometry, only whether the phagocytes cultured for the predetermined period have expressed a phagocytosis function is evaluated. For this reason, in the technology disclosed in the Non-Patent Literature, for example, it is difficult to evaluate a timing at which phagocytes cultured in the culture medium express the phagocytosis function, the number or the type of substances phagocytized by the phagocytes (phagocytized substances: an example of substances to which the function of a biological sample is applied) or the like.

In addition, in the technology disclosed in the Non-Patent Literature, it is also difficult to evaluate the influence of the environment in which phagocytes are cultured in a culture medium on the phagocytosis function. For example, it may be difficult to evaluate the environment that causes phagocytes to express the phagocytosis function. The environment includes, for example, the number of phagocytized substances existing around phagocytes or the concentration thereof, the number of phagocytes, a change in the shape of the phagocytes, the motility of the phagocytes, and the like. Since phagocytes obtained from a culture medium are introduced into a flow cytometer in flow cytometry, information regarding the culture environment of the culture medium of the phagocytes may be lost. Therefore, it is difficult to evaluate the culture environment related to expression of the phagocytosis function of the phagocytes.

Furthermore, although movements of phagocytes in a culture medium can be qualitatively observed using fluorescence imaging according to the technology disclosed in the Non-Patent Literature, quantitative evaluation of the movements is not disclosed. That is, quantitative evaluation of the expression of the phagocytosis function of phagocytes is difficult.

Therefore, the information processing system 1 according to the present embodiment detects a region (a region of interest) corresponding to an observation object or the like in at least one captured image among a plurality of captured images, calculates a feature amount related to a change in the region of interest appearing on the plurality of captured images, and calculates an evaluation value of the function of the observation object related to absorption or release such as a phagocytosis function on the basis of the calculated feature amount. Due to such technology, the function of the observation object related to absorption or release can be evaluated on the basis of movement of the observation object, a change in the shape thereof, or the like. Accordingly, while the function expressed by the observation object is observed, quantitative and temporal evaluation of the function is possible. Furthermore, the influence of the culture environment on the function of the observation object can be evaluated as well.

The overview of the information processing system 1 according to an embodiment of the present disclosure has been described above. The information processing device 20 included in the information processing system 1 according to an embodiment of the present disclosure is realized in the following embodiment. A specific configuration example and a process example of the information processing device 20 will be described below.

### <<2. Information processing device>>

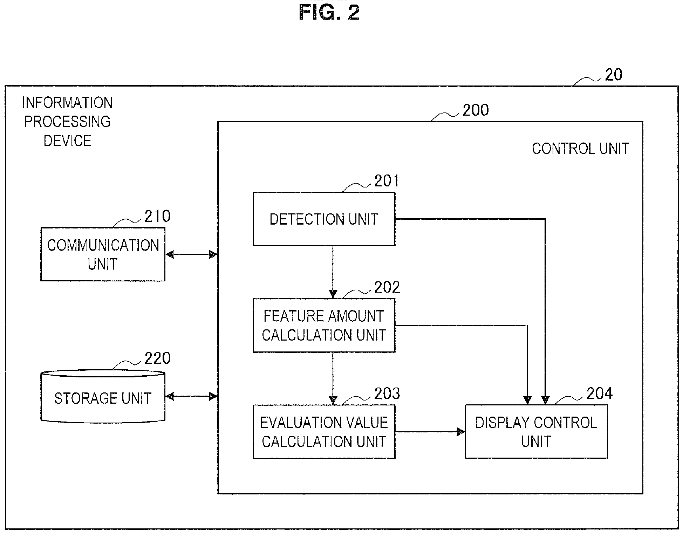

The information processing device 20 according to an embodiment of the present disclosure will be described below with reference to FIGS. 2 to 13. Note that, although the information processing device 20 according to the present embodiment will be described below as a technology for evaluating the phagocytosis function of an observation object such as phagocytes, a function to be evaluated is not limited to the phagocytosis function, and a function to be evaluated is not particularly limited as long as it is a function of an observation object related to absorption or release.

### <2.1. Configuration example>

FIG. 2 is a functional block diagram illustrating a functional configuration example of the information processing device 20 according to one embodiment of the present disclosure. As illustrated in FIG. 2, the information processing device 20 according to the present embodiment includes a control unit 200, a communication unit 210, and a storage unit 220. A function of the control unit 200 is implemented by a processing circuit such as a central processing unit (CPU) installed in the information processing device 20. Further, a function of the communication unit 210 is implemented by a communication device installed in the information processing device 20. Further, a function of the storage unit 220 is implemented by a storage device such as a storage installed in the information processing device 20. The respective function units will be described below.

### (Control unit)

The control unit 200 controls overall operations of the information processing device 20. In addition, the control unit 200 includes each of functions of a detection unit 201, a feature amount calculation unit 202, an evaluation value calculation unit 203, and a display control unit 204 as illustrated in FIG. 2 and dominantly controls operations of the information processing device 20 according to the present embodiment. The functions of the respective functional units included in the control unit 200 will be described below.

### (Communication unit)

The communication unit 210 is a communication means of the information processing device 20 and performs various kinds of communication with external devices through networks (or directly) in a wired or wireless manner. For example, the communication unit 210 communicates with the imaging device 10. More specifically, the communication unit 210 acquires a plurality of captured images generated by the imaging device 10. In the present embodiment, the communication unit 210 will be described as acquiring moving images generated by the imaging device 10. In addition, the communication unit 210 may communicate with devices other than the imaging device 10. For example, the communication unit 210 may transmit information regarding an evaluation value obtained by the evaluation value calculation unit 203, which will be described below, information regarding display of an evaluation value obtained by the display control unit 204 or the like to an external information processing device, display device, or the like.

### (Storage unit)

The storage unit 220 is a storage device installed in the information processing device 20 and stores information acquired by the communication unit 210, information obtained by the respective function units of the control unit 200, and the like. Further, the storage unit 220 appropriately outputs the stored information in response to a request from each function unit of the control unit 200 or from the communication unit 210.

Next, the functions of the respective function units installed in the control unit 200 will be described.

### (Detection unit)

The detection unit 201 detects at least one region of interest in one captured image constituting a moving image acquired by the communication unit 210 from the imaging device 10. Note that a region of interest in the present specification means a region for estimating a motion of an observation object.

The detection unit 201 may detect a region of interest in, for example, an image of an object included in one captured image. The region of interest may be a region corresponding to an observation object (e.g., a biological sample such as a phagocyte) included in a moving image (which will also be referred to as an observation object region) or a region corresponding to another object. The detection unit 201 may detect, for example, not only an observation object region but also a region of interest of an object other than an observation object. The object other than an observation object may be, for example, a substance to which the phagocytosis function of the observation object is applied (a phagocytized substance).

The detection unit 201 according to the present embodiment may detect, for example, a region surrounded by a closed curve forming a contour of an observation object and a phagocytized substance as a region of interest.

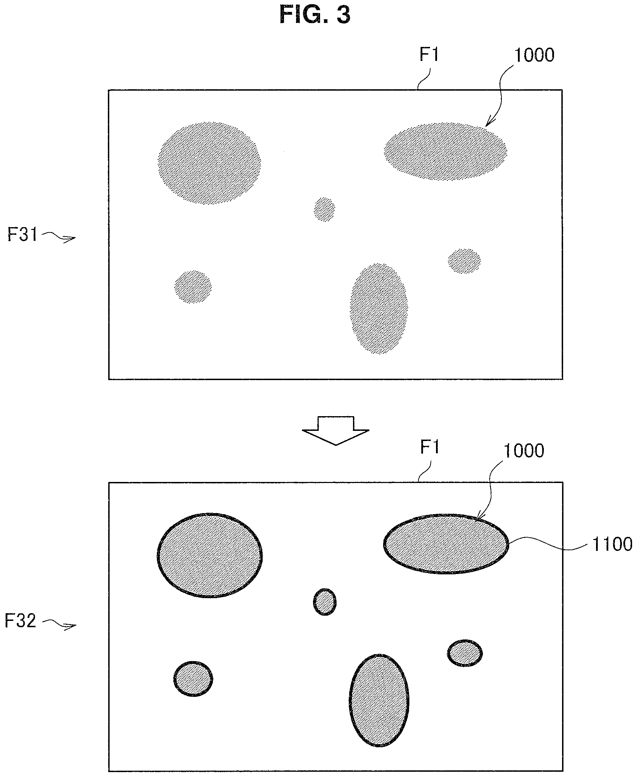

FIG. 3 is a diagram for describing a detection process of a region of interest performed by the detection unit 201 according to the present embodiment. First, it is assumed that a captured image F1 includes a plurality of object regions 1000 as indicated in a schematic diagram F31 of FIG. 3. The detection unit 201 may detect the object regions 100 through, for example, image recognition and set the object regions 1000 as regions of interest 1100 (refer to a schematic diagram F32 of FIG. 3). In this case, contour lines of the regions of interest 1100 may be contour lines of the object regions 1000 (i.e., the boundary lines between the object regions 1000 and non-object regions).

Note that the detection unit 201 may detect regions formed by closed curves corresponding to the contour lines of the objects as regions of interest as illustrated in FIG. 3, or may detect regions corresponding to tissues present inside the objects. More specifically, the regions of interest detected by the detection unit 201 may be regions corresponding to parts of tissues or the like included in an observation object. In a case in which parts of tissues included in the observation object are considered to express the phagocytosis function, for example, the detection unit 201 may detect regions corresponding to the parts of the tissues as regions of interest. Accordingly, the phagocytosis function of the tissues corresponding to desired regions can be evaluated. In addition, by reducing the sizes of the regions of interest to be as small as possible, calculation costs can be reduced.

In addition, the detection unit 201 may detect a plurality of regions in one captured image. In a case in which one captured image includes a plurality of objects, for example, the detection unit 201 may detect a region of interest with respect to each of the objects. Accordingly, a feature amount of a change in each of the objects (e.g., observation objects) can be calculated and the phagocytosis function of each of the observation objects can be evaluated. In addition, the detection unit 201 may also detect each of regions of interest in a plurality of captured images constituting a moving image. In this case, the feature amount calculation unit 202, which will be described below, can calculate a feature amount on the basis of a change in the respective detected regions of interest appearing among captured images even without estimating a change in the regions of interest.

In addition, a region of interest may be detected through an operation of a user using the information processing device 20 or the like, or the detection unit 201 may automatically detect a region of interest in one captured image constituting a moving image using a technique of image analysis or the like. In the latter case, the detection unit 201 may set a region corresponding to an object detected in the image analysis as a region of interest. The detection unit 201 may detect a region of interest using, for example, a feature amount related to a luminance of one captured image (e.g., dynamic range, etc.).

In addition, the above-described one captured image employed to detect a region of interest is not particularly limited as long as it is a captured image constituting a moving image. The above-described one captured image may be, for example, a captured image corresponding to the first frame of a moving image acquired by the communication unit 210. By setting a region of interest in the captured image of the first frame, for example, the position of the region of interest in the first frame can be used as a reference when a feature amount related to deformation of the region of interest in the moving image is calculated.

In addition, the above-described one captured image may be a captured image of a frame corresponding to a time point at which processing related to one kind of evaluation starts. The processing related to one kind of evaluation may be, for example, chemical processing of administering a medical agent to an observation object or the like. Accordingly, evaluation can be performed with reference to the moment immediately before the processing affects the observation object.

In addition, the detection unit 201 according to the present embodiment may dispose a plurality of tracking points for a region of interest detected in one captured image. Note that a tracking point in the present specification is a point disposed corresponding to a region of interest detected in one captured image. In the present embodiment, for example, tracking points are disposed on a contour line defining a region of interest at predetermined intervals. The feature amount calculation unit 202, which will be described below, detects positions of tracking points in another image captured at a different time point from in the one captured image used to detect the region of interest. The feature amount calculation unit 202 can detect a motion of the region of interest on the basis of movement positions of the tracking points.

In addition, the number of tracking points disposed and disposition intervals thereof may be decided according to the type of observation object or the shape of a region of interest. For example, when the shape of the region of interest significantly changes, it is desirable to increase the number of the tracking points disposed and reduce their disposition intervals. Accordingly, even if the form of an observation object significantly changes, the change in the form of the observation object can be tracked with high accuracy. In addition, in order to reduce a load of calculation, it is desirable to reduce the number of the tracking points disposed and increase their disposition intervals.

Furthermore, the detection unit 201 according to the present embodiment may identify detected regions of interest as a first region and a second region.

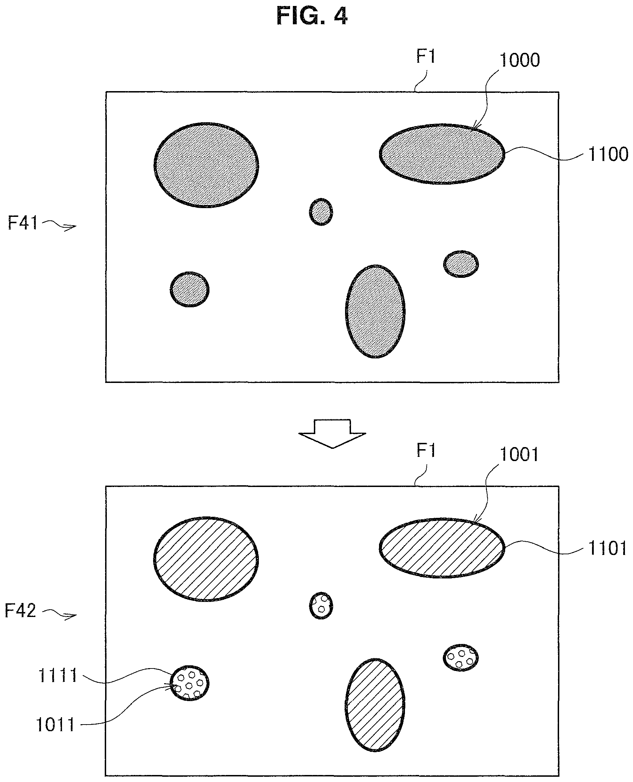

FIG. 4 is a diagram for describing an identification process of regions of interest performed by the detection unit 201 according to the present embodiment. As illustrated in the schematic diagram F41 of FIG. 4, in a case in which the detection unit 201 detects the regions of interest 1100 as the object regions 1000, the detection unit 201 identifies each of a first region 1101 and a second region 1111. That is, the detection unit 201 detects the regions of interest 1101 and 1111 with respect to an observation object region 1001 and a phagocytized substance region 1011 (refer to the schematic diagram F42 of FIG. 4).

As described above, since the detection unit 201 identifies whether each region of interest is a region of interest with respect to an observation object or a region of interest with respect to a phagocytized substance, the feature amount calculation unit 202, which will be described below, can calculate only a feature amount with respect to the observation object.

Note that, in a case in which the detection unit 201 can detect only either of the observation object and the phagocytized substance, for example, the above-described identification process may not be performed. In this case, the feature amount calculation unit 202 may calculate a feature amount with respect to either of the observation object and the phagocytized substance and the evaluation value calculation unit 203 may calculate an evaluation value on the basis of the calculated feature amount.

In addition, the identification process for a region of interest by the detection unit 201 may be performed on the basis of image information of the detected regions of interest. Here, the image information means information regarding the shape of the detected region of interest, internal pixel information of the region of interest, or the like. The information regarding a shape of the region of interest may be, for example, information regarding an area or a contour line length of the region of interest, or lengths thereof in the X and Y directions on a captured image. In addition, the internal pixel information of the region of interest may be internal color information of the region of interest (e.g., information regarding a specific fluorescent color exhibited by a fluorescence image) or texture information (e.g., pixel information obtained from a phase difference image or a bright field image of a captured image). Data related to such image information learned in advance may be stored in the storage unit 220 in advance, for example, in association with an observation object and a phagocytized substance, and the like. In this case, the detection unit 201 collates image information of the detected region of interest with the above-described learned data acquired from the storage unit 220. Accordingly, it is possible to identify whether the detected region of interest is a region of interest related to the observation object or a region of interest related to the phagocytized substance.

The information regarding the region of interest detected by the detection unit 201 is output to the feature amount calculation unit 202. In addition, the information regarding the region of interest may be output to the display control unit 204 for presentation to a user. Note that a region detected with respect to an observation object will be described as a region of interest in the following description unless specified otherwise.

### (Feature amount calculation unit)

The feature amount calculation unit 202 calculates a feature amount related to a change in a region of interest detected by the detection unit 201 appearing on a moving image (a change appearing on a plurality of captured images). The feature amount is used by the evaluation value calculation unit 203, which will be described below, to calculate an evaluation value. The types of feature amount used by the evaluation value calculation unit 203 to calculate an evaluation value may be one or many, and the number of selected types and a combination thereof are not particularly limited. The types of feature amount according to the present embodiment are as indicated in the following list.
(1) Feature amount based on a motion of a contour line of a region of interest
(2) Feature amount based on an internal motion of a region of interest
(3) Feature amount based on internal pixel information (luminance information) of a region of interest

Each of the feature amounts calculated by the feature amount calculation unit 202 will be described below.

### (1) Feature amount based on motion of contour line of region of interest

The feature amount calculation unit 202 may calculate a feature amount, for example, on the basis of a motion of a contour line of a region of interest on a moving image. A motion of a contour line means (a) a change in the position of the contour line or (b) a change in the shape of the contour line.

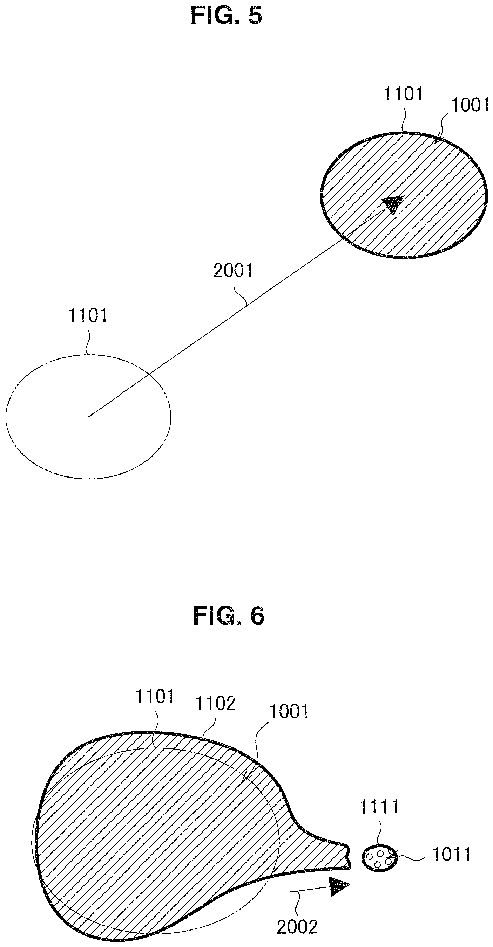

A change in the position of the contour line corresponds to movement of a region of interest made on a moving image. FIG. 5 is a diagram for describing a change in a position of a contour line of a region of interest. As illustrated in FIG. 5, the region of interest 1101 related to the observation object region 1001 is assumed to have moved to the position in another captured image denoted by the arrow 2001. At that moment, the observation object moves around to search for a phagocytized substance as illustrated in FIG. 5 in many cases. In this case, a contour line corresponding to the observation object also moves on the moving image in accordance with the movement of the observation object. Therefore, it can be assumed that the observation object is not phagocytizing a phagocytized substance when the region of interest is moving.

On the other hand, in a case in which the observation object is phagocytizing a phagocytized substance, the observation object remains stationary at a location in many cases. In this case, a contour line thereof may also remain stationary. Therefore, a feature amount based on a change in the position of the contour line is calculated, the feature amount is used to calculate an evaluation value, and thereby the presence or absence of expression of the phagocytosis function by the observation object or a timing thereof can be ascertained.

The feature amount based on a change in the position of the contour line may be calculated on the basis of, for example, a movement distance of a center position of the contour line. The center position may be specified using a weighted average of coordinates of the contour line or the like. In addition, the feature amount based on the change in the position of the contour line may be calculated using a known technology for calculating a movement distance of one region on a moving image.

In addition, a change in a shape of a contour line corresponds to local deformation of a contour line of a region of interest. FIG. 6 is a diagram for describing a change in the shape of the contour line of the region of interest. As illustrated in FIG. 6, a part of the shape of the contour line of the region of interest 1101 related to the observation object region 1001 is assumed to have been deformed in the direction indicated by the arrow 2002 in another captured image. At this time, the observation object may cause a part of a tissue to protrude in order to phagocytize a phagocytized substance existing in the vicinity of the observation object as illustrated in FIG. 6. Accordingly, the observation object captures the phagocytized substance using the protruding part. Then, in this case, the shape of the contour line of the region of interest corresponding to the observation object corresponding to the deformed part of the observation object also changes. Therefore, a feature amount based on the change in the shape of the contour line is calculated, the feature amount is used to calculate an evaluation value, and thereby the presence or absence of expression of the phagocytosis function by the observation object or a timing thereof can be ascertained.

The feature amount based on the change in the shape of the contour line may be calculated, for example, on the basis of an amount of change of an area of the region of interest or a length of the contour line. In addition, the feature amount may be calculated using a known technology for detecting a local change in the shape of the contour line of the region of interest.

Note that, in a case in which a feature amount based on the change in the contour line is calculated by the feature amount calculation unit 202, the feature amount calculation unit 202 may detect the change in the contour line of the region of interest using various techniques. For example, in a case in which the detection unit 201 disposes a plurality of tracking points in the region of interest, the feature amount calculation unit 202 may detect a motion of the region of interest by estimating motions of the tracking points disposed in the region of interest. A process of detecting a motion of the region of interest by the feature amount calculation unit 202 in the case in which a plurality of tracking points are disposed in the region of interest will be described below.

The feature amount calculation unit 202, at first, estimates positions of the tracking points that have been disposed in one captured image in another captured image of which the capturing time point is different from the one captured image. The other captured image may be a captured image of any frame among a few frames before and after the frame of the one captured image. The feature amount calculation unit 202 detects the motions of the tracking points in the dynamic image by performing a process for estimating positions of the tracking points in another captured image for respective captured images constituting the dynamic image. Further, the motion detected by the feature amount calculation unit 202 may be a motion in the entire dynamic image or a part of the dynamic image.

The feature amount calculation unit 202 may estimate positions of the tracking points based on, for example, a motion vector calculated by comparing a captured image to another captured image. This motion vector may be a motion vector calculated for each tracking point. The motion vector may be calculated using a known technique such as block matching, or a gradient method. The feature amount calculation unit 202 according to the present embodiment is described as estimating the motion vector using block matching.

For example, with regard to a tracking region in a predetermined size including tracking points, the feature amount calculation unit 202 may estimate positions of the tracking points in the other captured image by detecting a region of which information of pixels included in the tracking region of the captured image matches that of the other captured image from a predetermined block size (search range) of the other captured image. In this case, a size of the tracking region and the block size may be decided according to an imaging condition (for example, an imaging magnification) of the imaging device 10, the type of the observation object, the type of analysis performed on the observation object. When a movement of the observation object is large, for example, the tracking region or the block size may be set to be larger. Accordingly, accuracy in estimation of tracking points by the feature amount calculation unit 202 can be enhanced. In addition, when there are a number of tracking points for a region of interest, the tracking region or the block size may be adjusted to be small in order to reduce a load of calculation.

In addition, the feature amount calculation unit 202 may estimate a position of a tracking point in the other captured image generated at an imaging time point decided based on information of the observation object. When a change in the morphology of an observation object of which a speed of the change in the morphology is slow is tracked, for example, a difference in captured images between a plurality of consecutive frames generated by the imaging device 10 is small. For this reason, when a change in the shape of an observation object of which a speed of the change in the shape is slow is tracked, the feature amount calculation unit 202 may perform a detection process with a captured image a number of frames before or after the frame of the captured image as the other captured image. To be more specific, the feature amount calculation unit 202 may perform a detection process with a captured image a number frames after the captured image as the other captured image. The frame interval between the captured image and the other captured image enables the data amount of the captured image that is subject to a tracking process to be reduced. Accordingly, it is possible to reduce a load of calculation and track a motion of the region of interest over a long period of time. The frame interval can be appropriately set according to the type, a state, or the like of the observation object.

Note that the feature amount calculation unit 202 may calculate a feature amount based on the change in the position of the contour line or the change in the shape of the contour line on the basis of movement positions of detected tracking points. For example, the feature amount calculation unit 202 may calculate a statistical value such as an average value or a median of movement distances of a plurality of tracking points as a feature amount based on the change in the position of the contour line. In addition, the feature amount calculation unit 202 may calculate a feature amount based on the change in the shape of the contour line on the basis of a movement distance of a tracking point among a plurality of tracking points which is significantly longer than movement distances of other tracking points. Accordingly, the above-described feature amount can be calculated only using information obtained from the tracking points, and therefore calculation costs can be restricted.

Further, the feature amount calculation unit 202 may rearrange the tracking points for the region of interest after the motion detection. Accordingly, the estimation accuracy of the motion of the region of interest can be improved.

The detection process of a motion of the contour line of the region of interest and the calculation process of a feature amount using the tracking points performed by the feature amount calculation unit 202 in the case in which the tracking points are disposed in the region of interest have been described above. The present technology is not limited to the above example, and the detection process of a motion of the contour line of the region of interest may be performed using a known algorithm related to object tracking such as optical flow, pattern matching, or the like. In addition, a feature amount based on the motion of the contour line of the region of interest detected using such a known algorithm may be calculated by the feature amount calculation unit 202.

### (2) Feature amount based on internal motion of region of interest

In addition, the feature amount calculation unit 202 may calculate a feature amount on the basis of, for example, an internal motion in a region of interest made on a moving image. An internal motion of a region of interest is an internal motion of the region of interest on a moving image caused by a motion of an internal structure of an observation object corresponding to the region of interest.

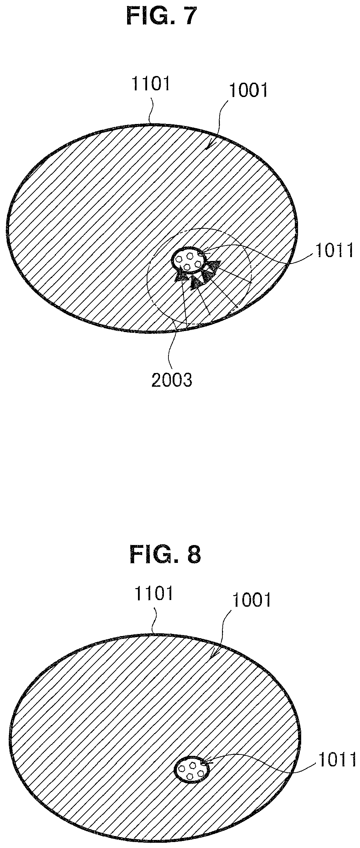

FIG. 7 is a diagram for describing an internal motion of the region of interest. The phagocytized substance region 1011 is assumed to exist inside the observation object region 1001 as illustrated in FIG. 7. At this time, the phagocytized substance is deemed to have been phagocytized by the observation object. In this case, since the phagocytized substance moves inside the observation object, internal motions of the observation object increase. For example, the increased motions can be detected in a neighboring region 2003 of the phagocytized substance region 1011 as illustrated in FIG. 7. In addition, when the observation object phagocytizes the phagocytized substance, a tissue inside the observation object may be caused to significantly move in order to digest the phagocytized substance. Then, internal motions of the region of interest corresponding to the observation object increase as well. Therefore, a feature amount based on the internal motions of the region of interest is calculated and the feature amount is used to calculate an evaluation value, and thereby the presence or absence of expression of the phagocytosis function by the observation object or a timing thereof can be ascertained.

Note that the feature amount calculation unit 202 may detect an internal motion vector of the region of interest as an internal motion of the region of interest. The motion vector may be a motion vector calculated for each of mesh squares by cutting the inside of the region of interest into mesh squares (mesh processing). In this case, a feature amount calculated by the feature amount calculation unit 202 may be a statistical value such as an average value, a median, a maximum value, a minimum value, or a standard deviation of sizes of motion vectors calculated for the respective mesh squares inside the region of interest. In addition, a size of a calculated motion vector itself may be used as a feature amount.

Note that the feature amount calculation unit 202 estimates a motion such as a change in a position and a shape of the region of interest on the moving image as described above. That is, the position and shape of the contour line of the region of interest may differ in respective captured images. For this reason, in a case in which the feature amount calculation unit 202 calculates a feature amount based on an internal motion of the region of interest, the feature amount calculation unit 202 may specify a position and a shape of a contour line of the region of interest for each captured image, detect a motion occurring inside the contour line, and calculate a feature amount based on the motion.

In addition, the feature amount may be a feature amount based on a motion occurring in a partial region inside the region of interest. In a case in which a pathway through which a phagocytized substance can pass such as the alimentary canal inside the observation object is known in advance, for example, a feature amount based on a motion of a region corresponding to the alimentary canal may be calculated. Such a region may be specified using a known technology related to image recognition or the like. Accordingly, the feature amount specialized for the motion related to the phagocytosis function can be calculated, and therefore, an evaluation value can be calculated with higher accuracy.

### (3) Feature amount based on internal pixel information (luminance information) of region of interest

In addition, the feature amount calculation unit 202 may calculate a feature amount on the basis of, for example, internal pixel information of the region of interest. Internal pixel information of the region of interest includes, for example, internal luminance information of the region of interest or an internal pattern of the region of interest. Such pixel information can change due to a motion of an internal structure of the observation object corresponding to the region of interest.

FIG. 8 is a diagram for describing internal pixel information of the region of interest. The inside of the observation object region 1001 in which the region of interest 1101 has been detected and the phagocytized substance region 1011 have different types of pixel information in most cases as illustrated in FIG. 8. Thus, for example, in a case in which pixels indicating a luminance or a pattern different from a luminance or a pattern corresponding to the observation object region 1001 exist within the region of interest 1101, there is a possibility of a foreign substance being incorporated into the observation object. In a case in which the above-described different luminance or pattern is a luminance or a pattern corresponding to the phagocytized substance region 1011, for example, there is a high possibility of the phagocytized substance being phagocytized by the observation object. Therefore, a feature amount based on internal pixel information of the region of interest is calculated, the feature amount is used to calculate an evaluation value, and thereby the presence or absence of expression of the phagocytosis function by the observation object or a timing thereof can be ascertained.

Note that the feature amount based on the pixel information may be, for example, a value related to a luminance of each pixel included in the region of interest (a statistical value such as an average, a minimum value, a maximum value, a median or a range of a luminance or a luminance gradient).

In addition, the feature amount based on the pixel information may be, for example, a feature amount based on a similarity of a pattern related to a texture of the observation object region. More specifically, in the case in which the phagocytized substance is phagocytized by the observation object, a similarity of an internal pattern of the region of interest to the pattern of the observation object region is considered to relatively decrease. Therefore, the similarity may be used as the feature amount based on the pixel information.

Feature amounts calculated by the feature amount calculation unit 202 have been described above. The feature amount calculation unit 202 calculates a feature amount related to a change in the region of interest on the moving image for the entire moving image or a partial section thereof. The calculation result of the feature amount is output to the evaluation value calculation unit 203. In addition, the calculation result of the feature amount may be output to the display control unit 204 to be presented to a user.

Note that, in the case in which the detection unit 201 identifies regions of interest as the first region and the second region, the feature amount calculation unit 202 may calculate a feature amount related to a change in the first region. In addition, as will be described in a modification example, the feature amount calculation unit 202 may calculate a feature amount related to a change in the second region. Specifically, the feature amount calculation unit 202 may calculate a feature amount related to a change in a position of a contour line of the second region. The calculated feature amount related to the change in the second region may be used to calculate an evaluation value by the evaluation value calculation unit 203 which will be described below. Accordingly, the phagocytosis function of the observation object based on a motion of the phagocytized substance can be evaluated.

In addition, in a case in which the detection unit 201 has detected regions of interest in a plurality of respective captured images constituting a moving image, the feature amount calculation unit 202, which will be described below, may calculate a feature amount on the basis of a change in the respective detected regions of interest appearing among the captured images. In this case, the feature amount calculation unit 202 may calculate a change in the region of interest on the moving image detected in one captured image constituting the moving image.

### (Evaluation value calculation unit)

The evaluation value calculation unit 203 calculates an evaluation value of the function of an observation object related to absorption or release (e.g., phagocytosis function) on the basis of one or a plurality of feature amounts calculated by the feature amount calculation unit 202. In the present embodiment, an evaluation value calculated by the evaluation value calculation unit 203 is, for example, (1) the number of times in which expression of the phagocytosis function by an observation object is found (2) a frequency of expression of the phagocytosis function of an observation object, and (3) an expression timing of the phagocytosis function by an observation object. With the number described in (1), it is possible to ascertain the number of observation objects that express the phagocytosis function. Accordingly, for example, a change in the phagocytosis function caused by administration of one or a plurality of medical agents can be evaluated. With the frequency of expression described in (2), it is possible to ascertain the number of phagocytized substances phagocytized by one observation object expressing the phagocytosis function. Accordingly, it is possible to quantitatively evaluate the presence or absence of expression of the phagocytosis function by an observation object as well as a specific frequency of expression of the function. In addition, with the expression timing described in (3), it is possible to ascertain a timing at which the phagocytosis function is expressed. Accordingly, temporal evaluation of the phagocytosis function of an observation object is possible. In the present embodiment, such an evaluation value related to the expression of the phagocytosis function can be calculated on the basis of a temporal change of a calculated feature amount. First, a method of determining expression of the phagocytosis function will be described.

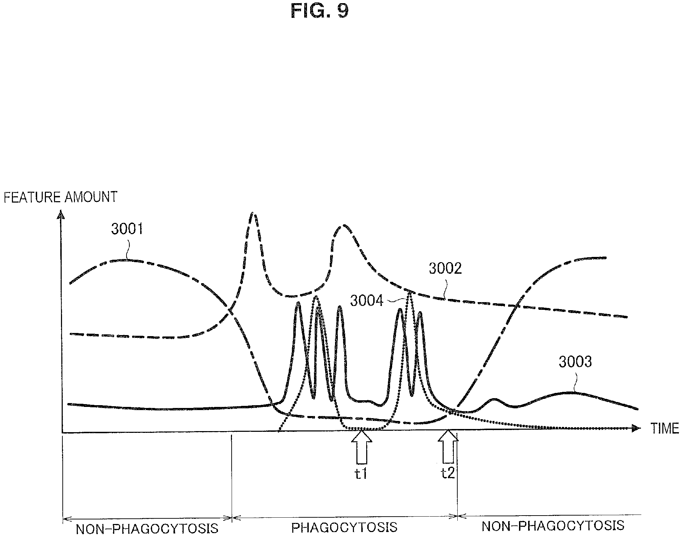

FIG. 9 is a graph illustrating an example of temporal change data of feature amounts calculated for an observation object. In the graph illustrated in FIG. 9, a movement amount curve 3001, a deformation amount curve 3002, an internal motion amount curve 3003, and a luminance curve 3004 are drawn.

The movement amount curve 3001 is a curve representing temporal change data of a feature amount related to a change in a position of a contour line of a region of interest. The deformation amount curve 3002 is a curve representing temporal change data of a feature amount related to a change in a shape of the region of interest. The internal motion amount curve 3003 is a curve representing temporal change data of a feature amount related to internal motions of the region of interest. In addition, the luminance curve 3004 is a curve representing temporal change data of a feature amount related to internal luminance information of the region of interest. Each of the feature amounts represented by these curves is a feature amount calculated by the feature amount calculation unit 202.

Referring to FIG. 9, the movement amount curve 3001 represents high values in non-phagocytosis sections (periods in which no phagocytosis function is expressed) of the observation object and represents low values in a phagocytosis section (a period in which the phagocytosis function is expressed) of the observation object. The reason for this is that the observation object relatively moves around when it does not express the phagocytosis function and the observation object stands still at a spot when it expresses the phagocytosis function. Therefore, the presence or absence of expression of the phagocytosis function can be determined on the basis of the degree of a feature amount represented by the movement amount curve 3001.

In addition, the deformation amount curve 3002 shows two peaks immediately before the phagocytosis section and in the phagocytosis section. These peaks are caused by the observation object changing a local shape thereof when it captures a phagocytized substance. In addition, since there are the two peaks as illustrated in FIG. 9, it is presumed that there are two instances of capturing phagocytized substances by the observation object. Therefore, it is possible to determine the presence or absence of expression of the phagocytosis function and calculate the frequency of expression of the phagocytosis function by using the peaks represented by the deformation amount curve 3002.

In addition, as is illustrated in FIG. 9, the feature amounts other than the peaks of the deformation amount curve 3002 in the phagocytosis section are expressed to a larger extent than the feature amounts of the deformation amount curve 3002 in the phagocytized section. The reason for this is that the observation object is enlarged by phagocytizing the phagocytized substances. The presence or absence of expression of the phagocytosis function can be determined by using this feature as well.

In addition, the internal motion amount curve 3003 shows a plurality of peaks in the phagocytosis section. These peaks are caused by motions made by the phagocytized substances incorporated into the observation object inside the observation object. The motions include, for example, motions of the observation object related to digestion of the phagocytized substances. Therefore, the presence or absence of expression of the phagocytosis function can be determined by using the peaks represented by the internal motion amount curve 3003.

In addition, the peaks may be divided into two group sections as illustrated in FIG. 9. This indicates that the observation object phagocytizes the phagocytized substances one by one in each of the group sections. Therefore, the frequency of expression of the phagocytosis function can be calculated from the number of groups. Furthermore, an end time of each of the group sections (e.g., a time t1 and a time t2 illustrated in FIG. 9) corresponds to a time at which the observation object ends phagocytosis of one phagocytized substance. Therefore, the evaluation value calculation unit 203 may calculate such a time as an evaluation value related to an expression timing of the phagocytosis function. The time is not limited to the end time of each group section, and may be a start time of each group section (a time at which the observation object starts phagocytizing a phagocytized substance). In summary, the presence or absence of expression of the phagocytosis function can be determined and a frequency of expression and an expression timing of the phagocytosis function can be calculated by using the peaks represented by the internal motion amount curve 3003.

In addition, the luminance curve 3004 shows a plurality of peaks in the phagocytosis section. The peaks are caused by a luminance of the phagocytized substances incorporated into the observation object. In a case in which a phagocytized substance is labeled with a fluorescent substance, for example, when the phagocytized substance is incorporated into the observation object, internal luminance information of the observation object changes due to fluorescence emitted by the fluorescent substance. Therefore, the presence or absence of expression of the phagocytosis function can be determined by using the peaks represented by the luminance curve 3004.

In addition, since there are two peaks of the luminance curve 3004 in the phagocytosis section as illustrated in FIG. 9, it is presumed that there are two instances of phagocytosis of phagocytized substances by the observation object. Therefore, the frequency of expression of the phagocytosis function can be calculated by using the peaks represented by the luminance curve 3004. In addition, the expression timing of the phagocytosis function can also be calculated on the basis of the positions of the two peaks.

As described above, the temporal change data of the feature amounts reflects the expression of the phagocytosis function by the observation object. Therefore, the phagocytosis function can be evaluated by analyzing the temporal change data of the feature amounts. For example, by determining the presence or absence of expression of the phagocytosis function of each observation object related to each region of interest on the basis of a feature amount of the region of interest, the evaluation value calculation unit 203 can calculate the number of observation objects that express the phagocytosis function as an evaluation value. The presence or absence of expression of the phagocytosis function can be determined by analyzing the temporal change data of the feature amounts. In addition, on the basis of the number or the positions of the peaks included in the temporal change data of the feature amounts, the evaluation value calculation unit 203 can calculate the frequency of expression of the phagocytosis function by the observation object or expression timings of the phagocytosis function as evaluation values. Accordingly, activation (or decline or malfunction) of the phagocytosis function of the observation object caused by administration of a medial agent, a reaction of the phagocytosis function to an administration timing of a medical agent, or the like can be evaluated. That is, evaluation on the phagocytosis function can be more detailed and diversified. Note that, for analysis of the temporal change data of the feature amounts by the evaluation value calculation unit 203, any of various known techniques related to data analysis such as peak detection or time-series clustering can be used.

Note that a single type or a plurality of types of temporal change data of feature amounts to be used in calculation of an evaluation value by the evaluation value calculation unit 203 may be used. For example, the evaluation value calculation unit 203 may calculate an evaluation value using a feature amount related to a change in a position of a region of interest, a feature amount related to an internal motion of the region of interest, and a feature amount related to internal luminance information of the region of interest. In a case in which an evaluation value is calculated using only one feature amount, there is even a possibility of a case in which a phagocytized substance simply slips through the top or bottom of an observation object being mistakenly ascertained as expression of the phagocytosis function by the observation object with respect to the phagocytized substance. The expression of the phagocytosis function by the observation object can be ascertained more reliably by using a combination of the plurality of feature amounts. As a result, accuracy in the evaluation value calculation can be improved.

In addition, the evaluation value calculation unit 203 may perform a gating process on a calculated feature amount or temporal change data of feature amounts. The gating process is a process of plotting data on one or a plurality of feature amounts related to observation objects in a dimension corresponding to the types of the feature amounts and sorting each of the plots into each group using a predetermined threshold value or the like. Accordingly, for example, the observation objects can be grouped in accordance with the presence or absence of expression of the phagocytosis function or the like, and the number or a proportion of observation objects that express the phagocytosis function can be easily calculated as an evaluation value.

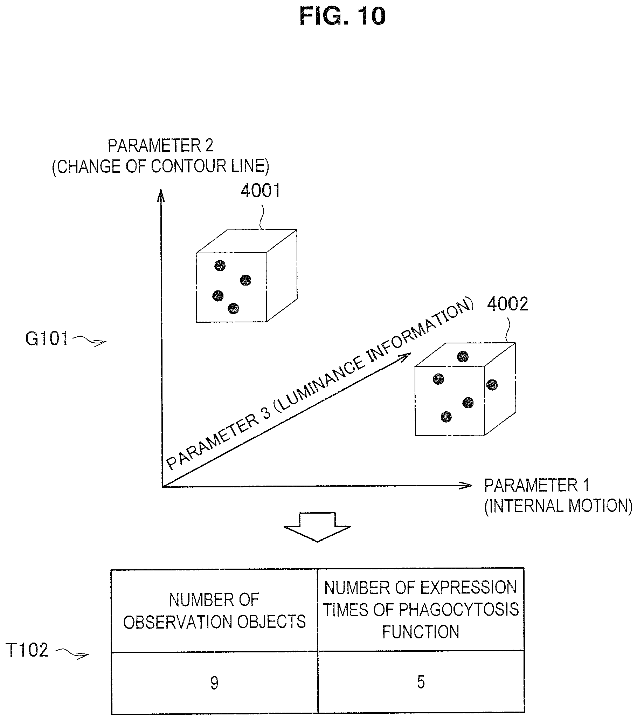

FIG. 10 is a diagram illustrating an example of the gating process performed by the evaluation value calculation unit 203 according to the present embodiment. As illustrated by the graph G101 of FIG. 10, an evaluation value related to an internal change (motion) in a region of interest may be used as a parameter 1, an evaluation value related to a change of a contour line of the region of interest may be used as a parameter 2, and an evaluation value related to luminance information of the region of interest may be used as a parameter 3 in the gating process. The parameter 1 may be, for example, the number of peaks (or the number of group sections) included in temporal change data of feature amounts related to the internal motion of the region of interest. In addition, the parameter 2 may be, for example, the total movement amount in the region of interest. In addition, the parameter 3 may be, for example, the number of peaks included in temporal change data of feature amounts related to internal luminance information of the region of interest.

As is illustrated by the graph G101, feature amounts each calculated for changes in the region of interest are plotted in the three-dimensional graph. Then, the evaluation value calculation unit 203 performs gating on the three-dimensional plotted graph and sets areas of interests 4001 and 4002. The plots included in the area of interest 4001 indicate high values of feature amounts related to the parameter 2 and low values of feature amounts related to the parameters 1 and 3. On the other hand, the plots included in the area of interest 4002 indicate low values of feature amounts related to the parameter 2 and high values of feature amounts related to the parameters 1 and 3.

From this indication, it is possible to determine that the observation objects corresponding to the plots included in the area of interest 4001 did not express the phagocytosis function and the observation objects corresponding to the plots included in the area of interest 4002 expressed the phagocytosis function. Therefore, the number of plots included in the area of interest 4002 are the number of observation objects that expressed the phagocytosis function, and the number is calculated by the evaluation value calculation unit 203 as an evaluation value (refer to Table T102 of FIG. 10; there are 5 out of 9 observation objects that expressed the phagocytosis function).

Note that the areas of interest 4001 and 4002 may be set through a user operation or automatically set to include plots satisfying predetermined conditions. The predetermined conditions may be appropriately adjusted in accordance with the phagocytosis function, a motility, and a culture environment of the observation objects, or the like. In addition, a size and a shape of the areas of interest are not particularly limited.

In addition, the number of types of feature amount to be used in the gating process or a combination thereof is not particularly limited. In addition an evaluation value calculated by the evaluation value calculation unit 203 in the gating process is not limited to the number or a proportion of the observation objects that express the phagocytosis function described above. For example, the evaluation value calculation unit 203 may calculate information regarding the number or a proportion of observation objects which have a similar frequency of expression or expression timing of the phagocytosis function of the observation objects or information regarding groups thereof through the gating process. More specifically, the evaluation value calculation unit 203 may perform grouping of observation objects having the same frequency of expression through the gating process. Accordingly, a trend in the expression of the phagocytosis function can be evaluated, and the observation objects showing different trends can also be compared.

Information regarding an evaluation value calculated by the evaluation value calculation unit 203 is output to the display control unit 204. In addition, the evaluation value calculation unit 203 may output a result of the gating process together with the evaluation value to the display control unit 204. Display control using a result of the gating process will be described below.

### (Display control unit)

The display control unit 204 causes a display device, which is not illustrated, or the like to display information regarding a result of a process performed by each function unit. The display control unit 204 according to the present embodiment may superimpose a region of interest detected by the detection unit 201 on a moving image as illustrated in, for example, FIG. 3 or FIG. 4. In addition, the display control unit 204 may cause feature amounts calculated by the feature amount calculation unit 202 to be displayed in a graph of temporal change as illustrated in FIG. 9.

In addition, the display control unit 204 may cause information regarding an evaluation value calculated by the evaluation value calculation unit 203 to be displayed. For example, the display control unit 204 may cause information regarding the number of observation objects that expressed the phagocytosis function, a frequency of expression of the phagocytosis function by the observation objects, or an expression timing of the phagocytosis function by the observation objects calculated as evaluation values to be displayed.

Furthermore, the display control unit 204 may control a display mode of a region of interest on the basis of a result of the gating process performed by the evaluation value calculation unit 203. A specific example thereof will be described below.

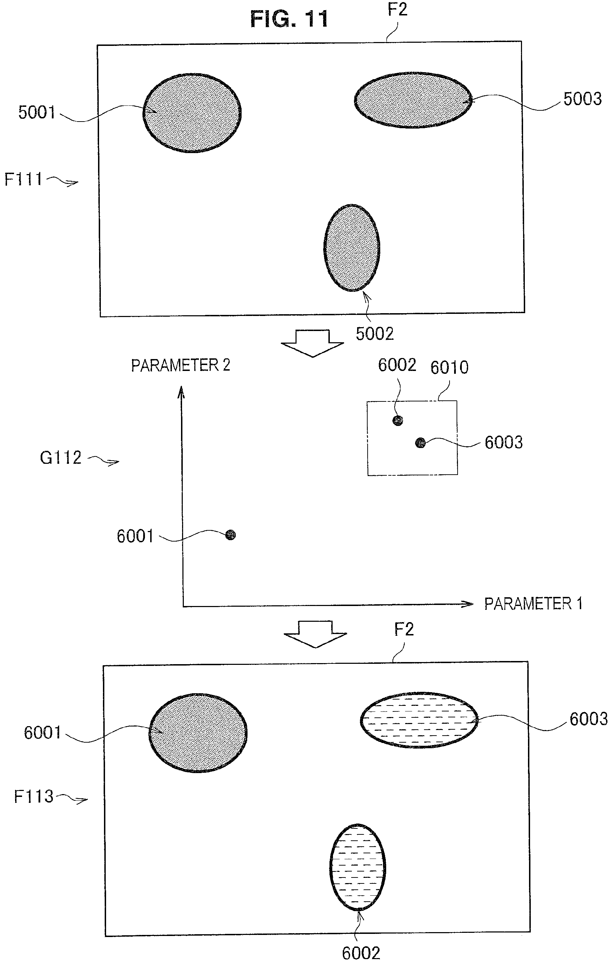

FIG. 11 is a diagram for describing an example of control of a display mode by the display control unit 204 based on a result of the gating process according to the present embodiment. As is illustrated in the schematic diagram Fill of FIG. 11, a captured image F2 is assumed to include regions of interest 5001 to 5003 (all of which correspond to observation object regions).

It is assumed that, for example, two feature amounts (corresponding to the parameter 1 and the parameter 2) of observation objects corresponding to the regions of interest 5001 to 5003 are each plotted in a two-dimensional graph through the gating process by the evaluation value calculation unit 203 (refer to the graph G112 of FIG. 11). More specifically, it is assumed that a plot 6001 of the observation object corresponding to the region of interest 5001, a plot 6002 of the observation object corresponding to the region of interest 5002, and a plot 6003 of the observation object corresponding to the region of interest 5003 are plotted at positions shown in the graph G112.

In this case, both the plot 6002 and the plot 6003 represent high values of both the parameter 1 and the parameter 2. On the other hand, the plot 6001 represents low values of both the parameter 1 and the parameter 2. When the observation objects are assumed to express the phagocytosis function in a case in which high values of the parameter 1 and the parameter 2 are shown, the observation objects corresponding to the plots 6002 and 6003 can be differentiated as expressing the phagocytosis function from the observation object corresponding to the plot 6001 not expressing the phagocytosis function.

Then, the display control unit 204 may perform control such that a display mode of the regions of interest 5002 and 5003 of the observation objects that express the phagocytosis function is different from that of the region of interest 5001 as a result of the gating process as illustrated the schematic diagram F113 of FIG. 11. Accordingly, the observation objects that express the phagocytosis function can be visualized.

In addition, the display control unit 204 may cause a graph showing gating results to be displayed as shown in the graph G112. In this case, not only the plots 6001 to 6003 but also an area of interest 6010 including the plots 6002 and 6003, for example, may be displayed as shown in the graph G112. In this case, the display control unit 204 may perform control such that a display mode of the regions of interest corresponding to the plots included in the area of interest 6010 in the display of the captured image F2 is different from that of another region of interest as illustrated in the schematic diagram F113.

In addition, the display control unit 204 may cause each of the plots plotted on the graph shown in the graph G112 or feature amounts related to one or the plurality of plots included in the area of interest 6010 to be displayed in a graph as illustrated in FIG. 9. In a case in which a plurality of plots are selected, a feature amount of each plot may be displayed, or a statistical value such as an average value, a median, a minimum value, or a maximum value of feature amounts of the plurality of selected plots may be displayed. Accordingly, it is possible to ascertain what event has occurred in expression of the phagocytosis function by the observation objects. Note that a size and a shape of the area of interest 6010 are not particularly limited.

As described above, by controlling the display mode of the region of interest using the result of the gating process, the display control unit 204 can visualize the evaluation result of expression of the phagocytosis function.

In addition, the display control unit 204 may perform control such that display modes of regions of interest differ in trends of the phagocytosis function by using results of the gating process. For example, the display control unit 204 may cause display modes of regions of interest to differ from each other in accordance with expression frequencies of the phagocytosis function by observation objects. In addition, the display control unit 204 may appropriately control display modes of regions of interest in accordance with evaluation values calculated by the evaluation value calculation unit 203. Accordingly, more detailed information can be visualized.

The display control process by the display control unit 204 has been described above. The display control by the display control unit 204 is appropriately executed through user operations or the like.

### <2.2. Process example>

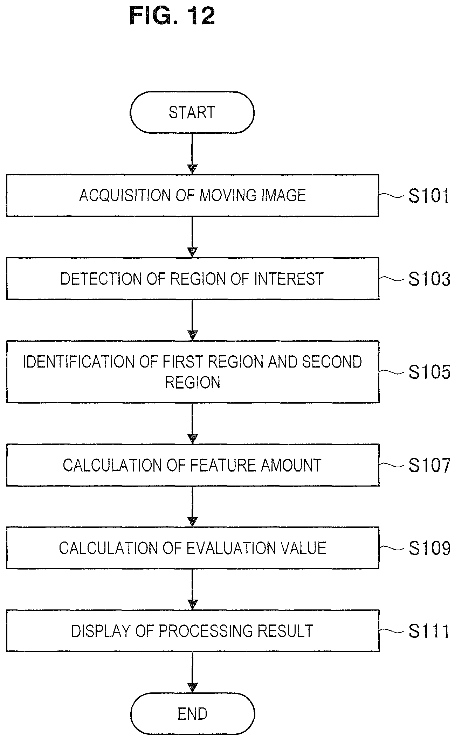

The configuration and the functions of the information processing device 20 according to the embodiment of the present disclosure have been described above. Next, an example of a process performed by the information processing device 20 according to the embodiment of the present disclosure will be described using FIG. 12.

FIG. 12 is a flowchart illustrating an example of a process performed by the information processing device 20 according to the embodiment of the present disclosure. First, the control unit 200 acquires moving image data from the imaging device 10 via the communication unit 210 (S101).

Next, the detection unit 201 extracts one captured image from the acquired moving image data and detects at least one region of interest in the one captured image (S103).

Next, the detection unit 201 identifies the detected regions of interest as a first region corresponding to an observation object and a second region corresponding to a phagocytized substance (S105).

Next, the feature amount calculation unit 202 calculates a feature amount related to a change in the region of interest (the first region) (S107). Next, the evaluation value calculation unit 203 calculates an evaluation value for the phagocytosis function on the basis of the feature amount calculated by the feature amount calculation unit 202 (S109).

Next, the display control unit 204 controls display of results processed by respective function units (Sill).

### <2.3. Effects>

The configuration example and the processing example of the information processing device 20 according to an embodiment of the present disclosure have been described above. The information processing device 20 according to the present embodiment calculates a feature amount related to a change in a detected region of interest and an evaluation value for the function of an observation object related to absorption or release such as the phagocytosis function on the basis of the calculated feature amount. With this configuration, a change in a form of the observation object or an internal motion thereof can be tracked and the presence or absence of expression of the phagocytosis function can be determined from characteristics of the change and motion. Accordingly, quantitative and temporal evaluation can be performed on the phagocytosis function of the observation object. In addition, the influence of a culture environment on the function of the observation object can be evaluated as well.

In addition, the information processing device 20 according to the present embodiment calculates feature amounts based on a change in a contour of a region of interest, an internal motion and internal luminance information of the region of interest as feature amounts. Accordingly, characteristic motions of an observation object when it expresses the phagocytosis function can be quantitatively ascertained, and thus the phagocytosis function can be evaluated with higher accuracy. In addition, the calculated feature amounts change in accordance with the characteristics of motions that are noted in the region of interest. That is, by calculating the plurality of feature amounts, motions made by the observation object related to the phagocytosis function can be ascertained in many aspects. Thus, by using the plurality of feature amounts in calculation of an evaluation value, accuracy in evaluation of the phagocytosis function can be further improved. Accordingly, for example, immune cells that are suitable for being using in a dendritic cell therapy or the like can be acquired on the basis of the above-described evaluation result. In addition, a change in the phagocytosis function of the observation object caused by administration of a medical agent can also be evaluated in more detail.

In addition, the information processing device 20 according to the present embodiment calculates an evaluation value on the basis of a temporal change of a feature amount. Accordingly, temporal evaluation on a phagocytosis function of an observation object, for example, evaluation on a frequency of expression of the phagocytosis function by the observation object and an expression timing thereof is possible. Therefore, subsequent evaluation can be performed simply not only on the presence or absence of expression of the phagocytosis function but also a temporal element related to the expression of the phagocytosis function or an event that can be caused by the expression of the phagocytosis function.

As described above, quantitatively and temporal evaluation can be performed on the phagocytosis function (an example of a function related to absorption or release) of an observation object included in a moving image with the information processing device 20 according to the present embodiment. Accordingly, the phagocytosis function can be evaluated in more detail with higher accuracy.

### <2.4. Modification example>

Although the information processing device 20 according to the above-described embodiment calculates a feature amount related to a change in a region of interest (a first region) related to an observation object with the feature amount calculation unit 202 and an evaluation value for the phagocytosis function on the basis of the feature amount with the evaluation value calculation unit 203, the present technology is not limited thereto. For example, the information processing device 20 may calculate a feature amount related to a change in a region of interest (a second region) related to a phagocytized substance with the feature amount calculation unit 202 and the above-described evaluation value using the feature amount with the evaluation value calculation unit 203.

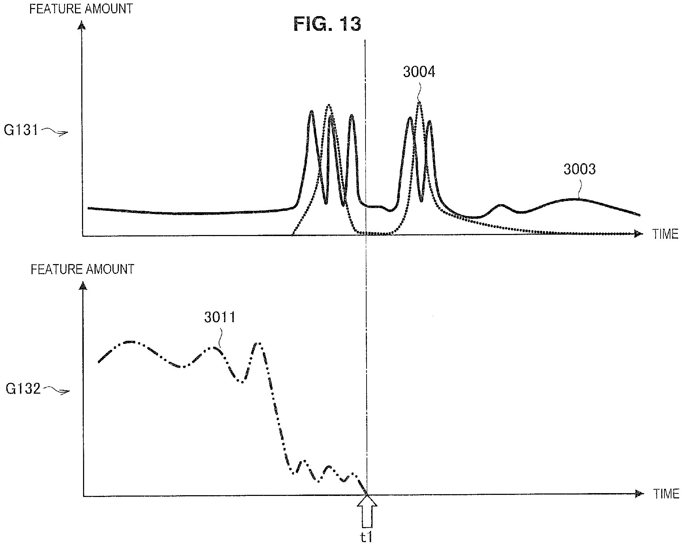

FIG. 13 illustrates examples of graphs showing temporal changes of feature amounts of a first region and a second region according to a modification example of the present embodiment. A graph G131 of FIG. 13 is a graph showing temporal change of feature amounts related to internal motions and internal luminance information of the first region, and a graph G132 of FIG. 13 is a graph showing change of a position of a contour line of the second region. An internal motion amount curve 3003 and a luminance curve 3004 shown in the graph G131 are the same as the internal motion amount curve 3003 and the luminance curve 3004 illustrated in FIG. 9. In addition, a curve 3011 shown in the graph G132 is a movement amount curve 3011 representing movement amounts of the second region.

The internal motion amount curve 3003 and the luminance curve 3004 exhibit peaks during phagocytosis of the observation object (the phagocytosis section) as illustrated in FIG. 9. At this time, it can be ascertained that feature amounts indicated by the movement amount curve 3011 are lower than before the phagocytosis. The reason for this is that motions of the phagocytized substance freely moving around the culture medium were restricted because it had been incorporated into the observation object. In addition, it is seen that the feature amount indicated by the movement amount curve 3011 at a time t1 is 0. The reason for this is that the phagocytized substance was digested by the observation object and thus the phagocytized substance disappeared. Therefore, it is considered that phagocytosis of the phagocytized substance by the observation object was completed at the time t1. This result matches, for example, the end of fluctuation of the internal motion amount curve 3003.

Therefore, by calculating an evaluation value using a feature amount related to a change in the second region (a temporal change of a feature amount), expression of the phagocytosis function of the observation object can be evaluated with higher accuracy.

Note that the feature amount calculation unit 202 may calculate only a feature amount related to a change in the second region, and the evaluation value calculation unit 203 may calculate an evaluation value on the basis of only the calculated feature amount. For example, it may be assumed that only one observation object is present in a culture medium and one or a plurality of phagocytized substances are present in the vicinity of the observation object. In this case, an evaluation value for the phagocytosis function of the observation object can be calculated in an observing manner by tracking a change in the region of interest (the second region) corresponding to the phagocytized substances without tracking a change in the region of interest (the first region) corresponding to the observation object.

The modification example of the information processing device 20 according to the embodiment of the present disclosure has been described above.

### <<3. Hardware Configuration example>>

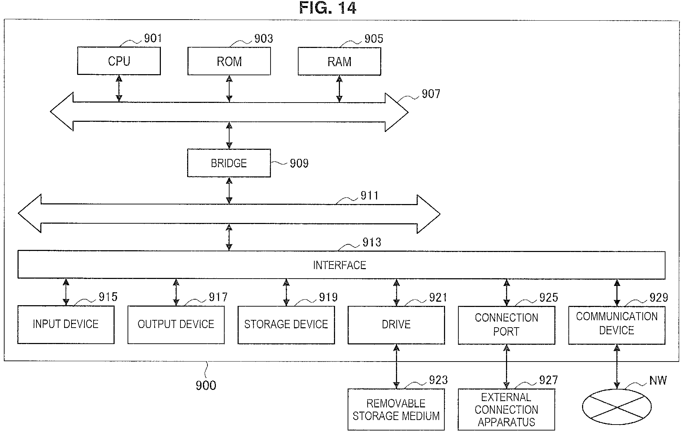

Next, with reference to FIG. 14, a hardware configuration of an information processing device according to an embodiment of the present disclosure is described. FIG. 14 is a block diagram showing a hardware configuration example of the information processing device according to the embodiment of the present disclosure. An illustrated information processing device 900 can realize the information processing device 20 in the above described embodiment.

The information processing device 900 includes a CPU 901, read only memory (ROM) 903, and random access memory (RAM) 905. In addition, the information processing device 900 may include a host bus 907, a bridge 909, an external bus 911, an interface 913, an input device 915, an output device 917, a storage device 919, a drive 921, a connection port 925, and a communication device 929. The information processing device 900 may include a processing circuit such as a digital signal processor (DSP) or an application-specific integrated circuit (ASIC), instead of or in addition to the CPU 901.

The CPU 901 functions as an arithmetic processing device and a control device, and controls the overall operation or a part of the operation of the information processing device 900 according to various programs recorded in the ROM 903, the RAM 905, the storage device 919, or a removable recording medium 923. For example, the CPU 901 controls overall operations of respective function units included in the information processing device 20 of the above-described embodiment. The ROM 903 stores programs, operation parameters, and the like used by the CPU 901. The RAM 905 transiently stores programs used when the CPU 901 is executed, and parameters that change as appropriate when executing such programs. The CPU 901, the ROM 903, and the RAM 905 are connected with each other via the host bus 907 configured from an internal bus such as a CPU bus or the like. The host bus 907 is connected to the external bus 911 such as a Peripheral Component Interconnect/Interface (PCI) bus via the bridge 909.

The input device 915 is a device operated by a user such as a mouse, a keyboard, a touchscreen, a button, a switch, and a lever. The input device 915 may be a remote control device that uses, for example, infrared radiation and another type of radio waves. Alternatively, the input device 915 may be an external connection device 927 such as a mobile phone that corresponds to an operation of the information processing device 900. The input device 915 includes an input control circuit that generates input signals on the basis of information which is input by a user to output the generated input signals to the CPU 901. The user inputs various types of data and indicates a processing operation to the information processing device 900 by operating the input device 915.

The output device 917 includes a device that can visually or audibly report acquired information to a user. The output device 917 may be, for example, a display device such as an LCD, a PDP, and an OELD, an audio output device such as a speaker and a headphone, and a printer. The output device 917 outputs a result obtained through a process performed by the information processing device 900, in the form of text or video such as an image, or sounds such as audio sounds.

The storage device 919 is a device for data storage that is an example of a storage unit of the information processing device 900. The storage device 919 includes, for example, a magnetic storage device such as a hard disk drive (HDD), a semiconductor storage device, an optical storage device, or a magneto-optical storage device. The storage device 919 stores therein the programs and various data executed by the CPU 901, and various data acquired from an outside. Further, the storage device 919 can realize the function of the storage unit 220 according to the above embodiments.

The drive 921 is a reader/writer for the removable recording medium 923 such as a magnetic disk, an optical disc, a magneto-optical disk, and a semiconductor memory, and built in or externally attached to the information processing device 900. The drive 921 reads out information recorded on the mounted removable recording medium 923, and outputs the information to the RAM 905. The drive 921 writes the record into the mounted removable recording medium 923.

The connection port 925 is a port used to directly connect devices to the information processing device 900. The connection port 925 may be a Universal Serial Bus (USB) port, an IEEE1394 port, or a Small Computer System Interface (SCSI) port, for example. The connection port 925 may also be an RS-232C port, an optical audio terminal, a High-Definition Multimedia Interface (HDMI (registered trademark)) port, and so on. The connection of the external connection device 927 to the connection port 925 makes it possible to exchange various kinds of data between the information processing device 900 and the external connection device 927.

The communication device 929 is a communication interface including, for example, a communication device for connection to a communication network NW. The communication device 929 may be, for example, a wired or wireless local area network (LAN), Bluetooth (registered trademark), or a communication card for a wireless USB (WUSB). The communication device 929 may also be, for example, a router for optical communication, a router for asymmetric digital subscriber line (ADSL), or a modem for various types of communication. For example, the communication device 929 transmits and receives signals in the Internet or transits signals to and receives signals from another communication device by using a predetermined protocol such as TCP/IP. The communication network NW to which the communication device 929 connects is a network established through wired or wireless connection. The communication network NW is, for example, the Internet, a home LAN, infrared communication, radio wave communication, or satellite communication. Further, at least one of the connection port 925 and the communication device 929 can realize the function of the communication unit 210 according to the above embodiments.

The example of the hardware configuration of the information processing device 900 has been introduced.

### <<4. Conclusion>>

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

For example, although the information processing system 1 is configured to be provided with the imaging device 10 and information processing device 20 in the above-described embodiment, the present technology is not limited thereto. For example, the imaging device 10 may have the function of the information processing device 20 (the detection function, the feature amount calculation function, and the evaluation value calculation function). In this case, the information processing system 1 is realized by the imaging device 10. In addition, the information processing device 20 may have the function of the imaging device 10 (imaging function). In this case, the information processing system 1 is realized by the information processing device 20. Further, the imaging device 10 may have a part of the function of the information processing device 20, and the information processing device 20 may have a part of the function of the imaging device 10.

In addition, although the information processing system 1 according to the above-described embodiment is described as a technology for evaluating the phagocytosis function of an observation object which is a phagocyte, the present technology is not limited thereto. For example, the information processing system according to the present technology can also evaluate a function related to release by an observation object, without being limited to the function related to absorption such as phagocytosis. More specifically, a function related to absorption and release of calcium ions by cells can also be evaluated. In this case, for example, a feature amount based on a motion made in a region near a contour of a cell may be calculated by the feature amount calculation unit, and an evaluation value for the function related to absorption and release of calcium ions may be calculated by the evaluation value calculation unit on the basis of the feature amount. Basically, absorption and release of calcium ions occur near cell membranes, and thus the function can be evaluated by ascertaining motions made in regions near the cell membranes.

In addition, although the information processing system 1 according to the above-described embodiment performs the process related to evaluation of the function of an observation object included in a moving image generated by the imaging device 10, the present technology is not limited thereto. For example, the information processing system 1 according to the present technology may perform the process related to evaluation of the function of an observation object included in a plurality of captured images having different imaging times. More specifically, the information processing system 1 according to the present technology may detect a region of interest with respect to the observation object included in a plurality of still images sequentially generated by the imaging device 10, calculate a feature amount on the basis of a change in the region of interest, and calculate an evaluation value for a function of the observation object on the basis of the feature amount. The plurality of captured images can be objects to be processed by the information processing system 1 according to the present technology as long as they are a plurality of captured images of a biological sample having different imaging times (which are sequentially generated).

The steps in the processes performed by the information processing device in the present specification may not necessarily be processed chronologically in the orders described in the flowcharts. For example, the steps in the processes performed by the information processing device may be processed in different orders from the orders described in the flowcharts or may be processed in parallel.

Also, a computer program causing hardware such as the CPU, the ROM, and the RAM included in the information processing device to carry out the equivalent functions as the above-described configuration of the information processing device can be generated. Also, a storage medium having the computer program stored therein can be provided.

Further, the effects described in this specification are merely illustrative or exemplified effects, and are not limitative. That is, with or in the place of the above effects, the technology according to the present disclosure may achieve other effects that are clear to those skilled in the art from the description of this specification.

### Reference Signs List

- 1: information processing system
- 10: imaging device
- 20: information processing device
- 200: control unit
- 201: detection unit
- 202: feature amount calculation unit
- 203: evaluation value calculation unit
- 204: display control unit
- 210: communication unit
- 220: storage unit

## Claims

1. An information processing device comprising:
a detection unit (201) configured to detect at least one region of interest in at least one captured image among a plurality of captured images of a biological sample having different imaging times;
a feature amount calculation unit (202) configured to calculate a feature amount related to a change in the at least one region of interest in the plurality of captured images; and
an evaluation value calculation unit (203) configured to calculate an evaluation value for a phagocytosis related function or a cell secretion function of the biological sample on a basis of the feature amount, wherein the feature amount calculation unit is configured to calculate the feature amount on a basis of a motion of a contour line of the at least one region of interest in the plurality of captured images.

2. The information processing device according to claim 1, wherein the feature amount on a basis of a motion of a contour line includes a feature amount related to a change in a position of the contour line.

3. The information processing device according to claim 1, wherein the feature amount on a basis of a motion of a contour line includes a feature amount related to a change in a shape of the contour line.

4. The information processing device according to claim 1, wherein the evaluation value calculation unit is configured to calculate a number of biological samples that express the function, as the evaluation value.

5. The information processing device according to claim 1, wherein the evaluation value calculation unit is configured to calculate a frequency of expression of the function by the biological sample, as the evaluation value.

6. The information processing device according to claim 1, wherein the evaluation value calculation unit is configured to calculate the evaluation value on a basis of a temporal change of at least one of the feature amounts.

7. The information processing device according to claim 6, wherein the evaluation value calculation unit is configured to calculate a timing at which the function is expressed by the biological sample, as the evaluation value.

8. The information processing device according to claim 1, wherein the evaluation value calculation unit is configured to perform gating on the feature amount and is configured to calculate the evaluation value on a basis of a result of the gating.

9. The information processing device according to claim 12, further comprising:
a display control unit configured to control a display mode of the region of interest on a basis of the result of the gating.

10. An information processing method of a processor, the information processing method comprising:
detecting at least one region of interest in at least one captured image among a plurality of captured images of a biological sample having different imaging times;
calculating a feature amount related to a change in the at least one region of interest in the plurality of captured images; and
calculating an evaluation value for a function related to phagocytosis or cell secretion of the biological sample on a basis of the feature amount, wherein the feature amount calculation calculates the feature amount on a basis of a motion of a contour line of the at least one region of interest in the plurality of captured images.

11. A program causing a computer to perform a method to claim 10.

## Patentansprüche

1. Datenverarbeitungsvorrichtung, die Folgendes umfasst:
eine Detektionseinheit (201), die konfiguriert ist, mindestens einen interessierenden Bereich in mindestens einem erfassten Bild unter mehreren erfassen Bildern einer biologischen Probe, die unterschiedliche Abbildungszeiten aufweisen, zu detektieren;
eine Merkmalsmengenberechnungseinheit (202), die konfiguriert ist, eine Merkmalsmenge bezüglich einer Änderung in dem mindestens einen interessierenden Bereich in den mehreren erfassten Bildern zu berechnen; und
eine Bewertungswertberechnungseinheit (203), die konfiguriert ist, einen Bewertungswert für eine Phagozytose-bezogene Funktion oder eine Zellsekretionsfunktion der biologischen Probe basierend auf der Merkmalsmenge zu berechnen, wobei die Merkmalsmengenberechnungseinheit konfiguriert ist, die Merkmalsmenge basierend auf einer Bewegung einer Konturlinie des mindestens einen interessierenden Bereichs in den mehreren erfassten Bildern zu berechnen.

2. Datenverarbeitungsvorrichtung nach Anspruch 1, wobei die Merkmalsmenge basierend auf einer Bewegung einer Konturlinie eine Merkmalsmenge bezüglich einer Änderung in der Position der Konturlinie enthält.

3. Datenverarbeitungsvorrichtung nach Anspruch 1, wobei die Merkmalsmenge basierend auf einer Bewegung einer Konturlinie eine Merkmalsmenge bezüglich einer Änderung in einer Form der Konturlinie enthält.

4. Datenverarbeitungsvorrichtung nach Anspruch 1, wobei die Bewertungswertberechnungseinheit konfiguriert ist, eine Zahl von biologischen Proben, die die Funktion ausdrücken, als den Bewertungswert zu berechnen.

5. Datenverarbeitungsvorrichtung nach Anspruch 1, wobei die Bewertungswertberechnungseinheit konfiguriert ist, eine Frequenz des Ausdrucks der Funktion durch die biologischen Proben als den Bewertungswert zu berechnen.

6. Datenverarbeitungsvorrichtung nach Anspruch 1, wobei die Bewertungswertberechnungseinheit konfiguriert ist, den Bewertungswert basierend auf einer zeitlichen Änderung von mindestens einer der Merkmalsmengen berechnet.

7. Datenverarbeitungsvorrichtung nach Anspruch 6, wobei die Bewertungswertberechnungseinheit konfiguriert ist, einen Zeitvorgabe, an der die Funktion durch die biologische Probe ausgedrückt wird, als den Bewertungswert zu berechnen.

8. Datenverarbeitungsvorrichtung nach Anspruch 1, wobei die Bewertungswertberechnungseinheit konfiguriert ist, auf der Merkmalsmenge ein Ausblenden durchzuführen, und konfiguriert ist, den Bewertungswert basierend auf einem Ergebnis des Ausblendens zu berechnen.

9. Datenverarbeitungsvorrichtung nach Anspruch 12, die ferner Folgendes umfasst:
eine Anzeigesteuereinheit, die konfiguriert ist, einen Anzeigemodus des interessierenden Bereichs basierend auf dem Ergebnis das Ausblendens zu steuern.

10. Datenverarbeitungsverfahren eines Prozessors, wobei das Datenverarbeitungsverfahren Folgendes umfasst:
Detektieren mindestens eines interessierenden Bereichs in mindestens einem erfassten Bild unter mehreren erfassten Bildern einer biologischen Probe, die unterschiedliche Abbildungszeiten aufweisen;
Berechnen einer Merkmalsmenge bezüglich einer Änderung in dem mindestens einen interessierenden Bereich in den mehreren erfassten Bildern; und
Berechnen eines Bewertungswertes für eine Funktion bezüglich Phagozytose oder Zellsekretion der biologischen Probe basierend auf der Merkmalsmenge, wobei die Merkmalsmengenberechnung die Merkmalsmenge basierend auf einer Bewegung einer Konturlinie des mindestens einen interessierenden Bereichs in den mehreren erfassen Bildern berechnet.

11. Programm, das einen Computer veranlasst, ein Verfahren nach Anspruch 10 durchzuführen.

## Revendications

1. Dispositif de traitement d'informations, comprenant :
une unité de détection (201) configurée pour détecter au moins une région d'intérêt dans au moins une image capturée parmi une pluralité d'images capturées d'un échantillon biologique ayant des temps de prise d'image différents ;
une unité de calcul de quantité de caractéristiques (202) configurée pour calculer une quantité de caractéristiques liée à un changement affectant l'au moins une région d'intérêt dans la pluralité d'images capturées ; et
une unité de calcul de valeur d'évaluation (203) configurée pour calculer une valeur d'évaluation pour une fonction liée à une phagocytose ou une fonction de sécrétion cellulaire de l'échantillon biologique sur la base de la quantité de caractéristiques, l'unité de calcul de quantité de caractéristiques étant configurée pour calculer la quantité de caractéristiques sur la base d'un mouvement d'une ligne de contour de l'au moins une région d'intérêt dans la pluralité d'images capturées.

2. Dispositif de traitement d'informations selon la revendication 1, dans lequel la quantité d'informations sur la base d'un mouvement d'une ligne de contour comporte une quantité de caractéristiques liée à un changement affectant une position de la ligne de contour.

3. Dispositif de traitement d'informations selon la revendication 1, dans lequel la quantité d'informations sur la base d'un mouvement d'une ligne de contour comporte une quantité de caractéristiques liée à un changement affectant une forme de la ligne de contour.

4. Dispositif de traitement d'informations selon la revendication 1, dans lequel l'unité de calcul de valeur d'évaluation est configurée pour calculer, comme valeur d'évaluation, un nombre d'échantillons biologiques exprimant la fonction.

5. Dispositif de traitement d'informations selon la revendication 1, dans lequel l'unité de calcul de valeur d'évaluation est configurée pour calculer, comme valeur d'évaluation, une fréquence d'expression de la fonction par l'échantillon biologique.

6. Dispositif de traitement d'informations selon la revendication 1, dans lequel l'unité de calcul de valeur d'évaluation est configurée pour calculer la valeur d'évaluation sur la base d'un changement temporel d'au moins une des quantités de caractéristiques.

7. Dispositif de traitement d'informations selon la revendication 6, dans lequel l'unité de calcul d'évaluation est configurée pour calculer, comme valeur d'évaluation, un instant auquel la fonction est exprimée par l'échantillon biologique.

8. Dispositif de traitement d'informations selon la revendication 1, dans lequel l'unité de calcul de valeur d'évaluation est configurée pour réaliser un filtrage sélectif sur la quantité de caractéristiques et est configurée pour calculer la valeur d'évaluation sur la base d'un résultat du filtrage sélectif.

9. Dispositif de traitement d'informations selon la revendication 12, comprenant en outre :
une unité de commande d'affichage configurée pour commander un mode d'affichage de la région d'intérêt sur la base du résultat du filtrage sélectif.

10. Procédé de traitement d'informations d'un processeur, le procédé de traitement d'informations comprenant :
la détection d'au moins une région d'intérêt dans au moins une image capturée parmi une pluralité d'images capturées d'un échantillon biologique ayant des temps de prise d'image différents ;
le calcul d'une quantité de caractéristiques liée à un changement affectant l'au moins une région d'intérêt dans la pluralité d'images capturées ; et
le calcul d'une valeur d'évaluation pour une fonction liée à une phagocytose ou une sécrétion cellulaire de l'échantillon biologique sur la base de la quantité de caractéristiques, le calcul de la quantité de caractéristiques calculant la quantité de caractéristiques sur la base d'un mouvement d'une ligne de contour de l'au moins une région d'intérêt dans la pluralité d'images capturées.

11. Programme amenant un ordinateur à réaliser un procédé selon la revendication 10.
